(19) Europäisches Patentamt
European Patent Office
Office européen des brevets

(11) Veröffentlichungsnummer: **0 008 748**
**B1**

(12) # EUROPÄISCHE PATENTSCHRIFT

(45) Veröffentlichungstag der Patentschrift: **25.03.81**

(51) Int. Cl.³: **C 07 C 13/43**, C 07 C 5/25

(21) Anmeldenummer: **79103097.6**

(22) Anmeldetag: **23.08.79**

(54) **Verfahren zur Herstellung von 5-Ethyliden-bicyclo-(2.2.1)-hept-2-en.**

(30) Priorität: **07.09.78 DE 2839000**

(43) Veröffentlichungstag der Anmeldung: **19.03.80 (Patentblatt 80/06)**

(45) Bekanntmachung des Hinweises auf die Patenterteilung: **25.03.81 Patentblatt 81/12**

(84) Benannte Vertragsstaaten: **BE FR GB IT NL**

(56) Entgegenhaltungen:
**DE - A - 1 668 190**
**DE - A - 1 768 733**
**DE - A - 2 149 703**
**CHEMICAL ABSTRACTS, Vol. 82, Nr. 13 31-03-1975, Seite 501, Nr. 86133y Columbus, Ohio, USA**

(73) Patentinhaber: **BUNAWERKE HLS GMBH**
**Postfach 1320**
**D-4370 Marl 1 (DE)**

(72) Erfinder: **Donike, Wilhelm, Dr.**
**Bitterfelder Strasse 7a**
**D-4370 Marl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 008 748 B1

Verfahren zur Herstellung von 5-Ethyliden-bicyclo-(2.2.1)-hept-2-en

5-Ethyliden-bicyclo-(2.2.1)-hept-2-en ist bekanntlich eines der technisch bedeutsamen nicht-konjugierten Diene, die mit Ethylen und mindestens einem weiteren α-Olefin, vornehmlich Propylen, polymerisiert werden, um ungesättigte, d.h. auch mit Schwefel oder Schwefelspendern vernetzbare Polyolefinkautschuke herzustellen. Dieser Einsatzzweck hat sicher dazu beigetragen, daß in den letzten Jahren eine Vielzahl von Verfahren zur Herstellung von 5-Ethyliden-bicyclo-(2.2.1)-hept-2-en entwickelt und publiziert wurden (US-PS 3 347 944, US-PS 3 535 395, DE-OS 16 68 189, DE-OS 16 68 190, DE-OS 25 09 262, DE-AS 21 49 703, FR-PS 15 69 488, SU-PS 435 222 und JP-AS 74/24 478). Allen diesen Verfahren ist gemeinsam, daß sie vom leicht durch eine sogenannte Diels-Alder-Reaktion aus Cyclopentadien-(1.3) und Butadien-(1.3) zugänglichen 5-Vinyl-bicyclo-(2.2.1)-hept-2-en ausgehen, das dann mit Hilfe eines speziellen Isomerisierungskatalysators mehr oder weniger in 5-Ethyliden-bicyclo-(2.2.1)-hept-2-en überführt wird. Bei sämtlichen bekannten Verfahren bestehen jedoch Nachteile, die beispielsweise in schlechten Raum-Zeit-Ausbeuten und relativ hohem Katalysatorverbrauch bei niedrigen Reaktionstemperaturen (<100° C) liegen oder darin, daß bei höheren Temperaturen (>100° C) größere Mengen 4.5.8.9-Tetrahydroinden als Nebenprodukt entstehen.

Aufgabe der vorliegenden Erfindung war es daher, ein Verfahren zur Herstellung von 5-Ethyliden-bicyclo-(2.2.1)-hept-2-en zu entwickeln, das bei relativ tiefen Temperaturen und mit geringen Katalysatormengen zu hohen Raum-Zeit-Ausbeuten führt.

Diese Aufgabe wurde nun durch das in den Ansprüchen beschriebene Verfahren gelöst.

Die Isomerisierungsreaktion von 5-Vinyl-bicyclo-(2.2.1)-hept-2-en in 5-Ethyliden-bicyclo-(2.2.1)-hept-2-en läßt sich durch folgende Formelgleichung wiedergeben :

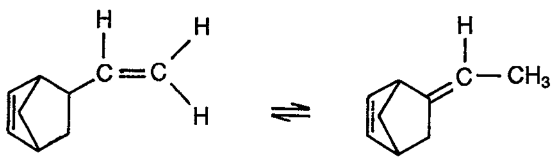

Das als Ausgangsprodukt dienende 5-Vinyl-bicyclo-(2.2.1)-hept-2-en kann nach bekannten Verfahren des Standes der Technik leicht durch eine sogenannte Diels-Alder-Reaktion aus Cyclopentadien-(1.3) und Butadien-(1.3) erhalten werden (DE-AS 19 10 566 ; A.F. Plate und N.A. Belikova in Zhurnal Obshchei Khimii (1960), *30,* Nr. 12, 3945-53).

Bei der Durchführung des Verfahrens gemäß der Erfindung ist es nicht wesentlich, daß das 5-Vinyl-bicyclo-(2.2.1)-hept-2-en absolut rein ist, jedoch ist zur Erzielung guter Ergebnisse die Anwesenheit zu großer Mengen an Verunreinigungen zu vermeiden.

Die Isomerisierung kann gegebenenfalls in einem inerten organischen Lösemittel durchgeführt werden. Dabei können gegebenenfalls substituierte aliphatische, cycloaliphatische und aromatische Lösemittel, wie z.B. Pentan, Hexan, Heptan, Cyclohexan, Benzol und Toluol Verwendung finden. Aromatische Lösemittel, vornehmlich Benzol, sind bevorzugt. Bei Verwendung eines Lösemittels kann die Konzentration des 5-Vinyl-bicyclo-(2.2.1)-hept-2-en innerhalb weiter Grenzen variiert werden, ohne die Isomerisierung zu beeinträchtigen. Im allgemeinen liegt das Gewichtsverhältnis von Lösemittel zu 5-Vinyl-bicyclo-(2.2.1)-hept-2-en im Bereich von 10 : 1 bis 0,1 : 1, vorzugsweise 5 : 1 bis 0,5 : 1.

Als Isomerisierungskatalysator wird beim erfindungsgemäßen Verfahren Eisen-pentacarbonyl, $Fe(CO)_5$, verwendet, das bekanntlich durch Erhitzen von fein verteiltem Eisen mit Kohlenmonoxid unter Druck auf 150 bis 200° C erhalten werden kann.

Im allgemeinen wird das Eisen-pentacarbonyl in einer Menge von 0,005 bis 10, vorzugsweise von 0.1 bis 1,5 Gewichtsprozent, bezogen auf 5-Vinyl-bicyclo-(2.2.1)-hept-2-en, eingesetzt.

Die katalytische Wirksamkeit des Eisen-pentacarbonyls wird bei dem erfindungsgemäben Verfahren durch die gleichzeitige Zugabe eines Amins als Cokatalysator noch gesteigert. Geeignet sind dabei primäre, sekundäre oder tertiäre Amine, vorzugsweise aliphatische Amine mit 2 bis 18 C-Atomen, wie beispielsweise Di-n-butylamin. Das Amin wird in einer Menge von 0,01 bis 5 Gewichtsprozent, bezogen auf eingesetztes 5-Vinyl-bicyclo-(2.2.1)-hept-2-en zugesetzt.

Neben den aufgeführten Cokatalysatoren können dem Reaktionsansatz, vornehmlich dann, wenn er eine technisch interessante Größe besitzt, mit Vorteilen noch Inhibitoren, z.B. aus der Gruppe der Chinone und Hydrochinone, zugesetzt werden. Die Inhibitoren werden gegebenenfalls in einer Menge von 10 bis 1 000 Gewichts-ppm, bezogen auf eingesetztes 5-Vinyl-bicyclo-(2.2.1)-hept-2-en eingesetzt.

Zum Bestrahlen kann jede Ultraviolett-Strahlquelle verwendet werden, die Strahlen der Wellenlänge $7,8.10^{-4}$ mm bis $1.10^{-5}$ mm abstrahlt. Besonders bevorzugt ist der Wellenlängenbereich von 2 bis $4.10^{-4}$ mm, d.h. eine Strahlung, wie sie von den handelsüblichen Ultraviolett-lampen erzeugt wird. Den Effekt, den die Strahlungsquelle auf den Isomerisierungskatalysator und damit auf die Isomerisierung ausübt, läßt sich durch die Zahl der verwendeten Strahlungsquellen, durch die Zeit der Einwirkung der Strahlung auf das Reaktionsgemisch und die räumliche Entfernung des Reaktionsgemisches von der Strahlungsquelle steuern.

Ein besonderer Vorteil des erfindungsgemäßen Verfahrens liegt darin, das es bei relativ niedrigen Temperaturen durchgeführt werden kann, so daß keine Nebenprodukte durch Umlagerung des 5-Ethyliden-bicyclo-(2.2.1)-hept-2-en zu 4.5.8.9-Tetrahydroinden und/oder durch Polymerisation des 5-Vinyl-bzw. 5-Ethyl-bicyclo-(2.2.1)-hept-2-ens entstehen. Im allgemeinen erfolgt die Isomerisierung bei Temperaturen von 40 bis 100° C, vorzugsweise von 50 bis 90° C. Besonders bevorzugt ist der Temperaturbereich von 75 bis 85° C.

Das erfindungsgemäße Verfahren kann sowohl kontinuierlich als auch diskontinuierlich durchgeführt werden.

Im Normalfall wird bei Atmosphärendruck gearbeitet, aber auch andere Druckverhältnisse (Überdruck, Unterdruck) sind möglich.

Das erfindungsgemäße Verfahren stellt sich im allgemeinen wie folgt dar :

5-Vinyl-bicyclo-(2.2.1)-hept-2-en wird gegebenenfalls mit einem Lösemittel im Reaktionsgefäß vorgelegt und mit Eisenpentacarbonyl als Katalysator und einem Amin als Cokatalysator versetzt.

Diese Mischung wird unter Rühren und Bestrahlung mit einer UV-Lampe auf Reaktionstemperatur erhitzt und gehalten.

Zur Kontrolle des Umsatzes werden kleine Probemengen gaschromatographisch untersucht. Der Versuch wird durch Kühlen des Reaktionsgemisches abgebrochen, wenn ein ausreichender Umsatz festgestellt wird.

Die Aufarbeitung des Reaktionsgemisches erfolgt durch Vakuumdestillation. Das Destillat wird gaschromatographisch untersucht. Eine weitere Reinigung des Produktes ist nicht erforderlich.

Das nach dem erfindungsgemäßen Verfahren hergestellte 5-Ethyliden-bicyclo-(2.2.1)-hept-2-en wird — wie bereits erwähnt — vornehmlich mit Ethylen und mindestens einem weiteren α-Olefin zu ungesättigten Polyolefinkautschuken copolymerisiert.

Das erfindungsgemäße Verfahren wird anhand der nachfolgenden Beispiele näher erläutert :

## Beispiel 1

Die Analysen wurden mit einem Gaschromatographen der Firma Perkin-Elmer F 22 (FID) durchgeführt, dessen 5-m-Säule mit 10 % Polypropylenglykol 600 auf Kieselgur gefüllt war (Trägergas $H_2$, 1.5 bar, Säulentemperatur 100° C).

In einem mit Rückflußkühler, Stickstoffeinleitungsrohr und Rührer versehenen Quarzkolben wird eine Mischung von 100 g Benzol, 100 g 5-Vinyl-bicyclo-(2.2.1)-hept-2-en, 1 g Dibutylamin und 1 g Eisenpentacarbonyl unter Rühren und Bestrahlung mit einer UV-Lampe von 150 Watt Leistung, 366 nm, auf 80° C erhitzt. Nach einer Stunde wurde eine Probe entnommen und gaschromatographisch untersucht, wobei das Verhältnis von 5-Ethyliden-bicyclo-(2.2.1)-hept-2-en zu 5-Vinyl-bicyclo-(2.2.1)-hept-2-en 82 : 18 betrug ; nach zwei Stunden lag das Verhältnis bei 97 : 3. Das Reaktionsgemisch wurde abgekühlt und rektifiziert. Die gaschromatographische Analyse des Destillats ergab 96,5 g 5-Ethyliden-bicyclo-(2.2.1)-hept-2-en und 2,5 g 5-Vinyl-bicyclo-(2.2.1)-hept-2-en. Nebenprodukte der Reaktion waren gaschromatographisch nicht nachweisbar.

## Beispiele 2-6

In der in Beispiel 1 beschriebenen Apparatur wurde jeweils 100 g 5-Vinyl-bicyclo-(2.2.1)-hept-2-en unter den in Tabelle 1 angegebenen Bedingungen umgesetzt.

Die Ausbeuten an 5-Ethyliden-bicyclo-(2.2.1)-hept-2-en zeigt die letzte Spalte der Tabelle 1.

Die Analysen wurden mit dem im Beispiel 1 angeführten Gaschromatographen durchgeführt.

TABELLE 1

| Beispiel Nr. | Katalysator $Fe(CO)_5$ Gew.-% | Cokatalysator Gew.-% | Reaktions-temperatur °C | Lösemittel g | Reaktionszeit h | Umlagerung Gew.-% |
|---|---|---|---|---|---|---|
| 2 | 1 | Dibutylamin 1 | 71-74 | Benzol 100 | 3 | 97.0 |
| 3 | 1 | Dibutylamin 1 | 85 | Toluol 200 | 2 | 95.5 |
| 4 | 1 | Dibutylamin 1 | 80 | Cyclohexan 100 | 2 | 97.2 |
| 5 | 1 | Diphenylamin 1 | 75 | Benzol 100 | 2 | 97.5 |
| 6 | 0.5 | Dibutylamin 0.5 | 75 | Benzol 100 | 2 | 97.0 |

**Ansprüche**

1. Verfahren zur Herstellung von 5-Ethyliden-bicyclo-(2.2.1)-hept-2-en durch Isomerisierung von 5-Vinyl-bicyclo-(2.2.1)-hept-2-en gegebenenfalls in Gegenwart eines organischen Lösemittels mit Hilfe eines Katalysators bestehend aus Eisenpentacarbonyl und einem Amin,
dadurch gekennzeichnet,
daß man die Isomerisierung unter Einwirkung von UV-Strahlen bei Temperaturen von 40 bis 100° C durchführt.

2. Verfahren nach Anspruch 1,
dadurch gekennzeichnet,
daß man die Isomerisierung bei Temperaturen von 50 bis 90° C durchführt.

3. Verfahren nach Anspruch 2,
dadurch gekennzeichnet,
daß man die Isomerisierung bei Temperaturen von 75 bis 85° C durchführt.

**Claims**

1. Process for the production of 5-ethylidene-bicyclo-(2.2.1)-hept-2-ene by isomerization of 5-vinyl-bicyclo-(2.2.1)-hept-2-ene, in the presence of an organic solvent, as the case may be, with the aid of a catalyst being composed of iron pentacarbonyl and an amine,
characterized in that
isomerization is effected under exposure to UV radiation at temperatures of between 40 and 100° C.

2. Process according to claim 1,
characterized in that
isomerization is effected at temperatures of between 50 and 90° C.

3. Process according to claim 2,
characterized in that
isomerization is effected at temperatures of between 75 and 85° C.

**Revendications**

1. Procédé de fabrication d'éthylidène-5-bicyclo-[2.2.1]-heptène-2 par isomérisation de vinyle-5-bicyclo-[2.2.1]heptène-2, le cas échéant en présence d'un solvant organique, moyennant un catalyseur se composant de fer-penta-carbonyle et d'une amine,
caractérisé par le fait que
l'isomérisation s'effectue sous l'action de rayons U.V. à des températures comprises entre 40 et 100° C.

2. Procédé selon revendication 1,
caractérisé par le fait que
l'isomérisation s'opère à des températures comprises entre 50 et 90 °C.

3. Procédé selon revendication 2,
caractérisé par le fait que
l'isomérisation se fait à des températures comprises entre 75 et 85 °C.